# EUROPÄISCHE PATENTSCHRIFT

(11) **EP 1 626 726 B1**
(45) Veröffentlichungstag und Bekanntmachung des Hinweises auf die Patenterteilung: **13.09.2006**
(21) Anmeldenummer: 04734972.5
(22) Anmeldetag: 27.05.2004
(51) Int. Cl.: A61K 31/54, A61K 31/4439, A61P 25/28, A61P 9/10

(54) **VERWENDUNG VON LORNOXICAM ODER LORNOXICAM-ANALOGA VERBINDUNGEN**
USE OF LORNOXICAM OR LORNOXICAM ANALOGUE COMPOUNDS
UTILISATION DE COMPOSES DU LORNOXICAM OU LORNOXICAM-ANALOGUES

(30) Priorität: 27.05.2003 AT 8192003
(43) Veröffentlichungstag der Anmeldung: 22.02.2006
(73) Patentinhaber: Binder, Eva HF, 1190 Wien (AT)
(72) Erfinder: BINDER, Dieter DI, deceased (AT)
(74) Vertreter: Sonn & Partner Patentanwälte
(86) Internationale Anmeldenummer: PCT/AT2004/000185
(87) Internationale Veröffentlichungsnummer: WO 2004/105766

(56) Entgegenhaltungen:
- AT-B- 400 437
- US-A1- 2003 225 054
- US-B1- 6 429 223
- DATABASE EMBASE [Online] ELSEVIER SCIENCE PUBLISHERS, AMSTERDAM, NL; 29. August 2002 (2002-08-29), ULLRICH T ET AL: "Conformationally constrained nicotines: Polycyclic, bridged, and spiro-annulated analogues as novel ligands for the nicotinic acetylcholine receptor" XP002298059 Database accession no. EMB-2002330335 & JOURNAL OF MEDICINAL CHEMISTRY 29 AUG 2002 UNITED STATES, Bd. 45, Nr. 18, 29. August 2002 (2002-08-29), Seiten 4047-4054, ISSN: 0022-2623
- DATABASE MEDLINE [Online] US NATIONAL LIBRARY OF MEDICINE (NLM), BETHESDA, MD, US; 30. August 2002 (2002-08-30), AVRAMOVICH YAEL ET AL: "Non-steroidal anti-inflammatory drugs stimulate secretion of non-amyloidogenic precursor protein." XP002298060 Database accession no. NLM12070143 & THE JOURNAL OF BIOLOGICAL CHEMISTRY. 30 AUG 2002, Bd. 277, Nr. 35, 30. August 2002 (2002-08-30), Seiten 31466-31473, ISSN: 0021-9258

## Beschreibung

Die Erfindung betrifft die Herstellung von pharmazeutischen Zusammensetzungen zur Behandlung der Alzheimer'schen Erkrankung und der Arteriosklerose.

Die Ätiologie der Alzheimer'schen Erkrankung (AE) ist bisher nicht geklärt. Gemäß der "Amyloid-Hypothese" der AE kommt es zu einer Veränderung in der Spaltung des Amyloidvorläuferproteins (APP). Das sogenannte β-42-Peptid wird abgelagert, wodurch sich cerebrale Plaques bilden. Als Folge auch der resultierenden Hypoperfusion kommt es zur neuronalen Degeneration.

Die gegenwärtig angewendeten Behandlungsmethoden umfassen alle die Verabreichung cholinerger Agentien, insbesondere Inhibitoren der Acetylcholinesterase, da AE mit wesentlichen Verlusten cholinerger Neuronen verbunden ist und Acetylcholinesterase-Inhibitoren den Acetylcholin-Spiegel erhöhen, so dass die verbleibenden Neuronen aktiviert bleiben ("feuern"). Mit dieser Behandlung kann leider der progressive Verlust an Neuronen nicht gestoppt werden.

Weitere Zielmoleküle, deren Beeinflussung derzeit in Zusammenhang mit einer AE-Therapie diskutiert und erprobt wird, sind Secretase-modulierende Stoffe, insbesondere β- und γ-Secretase-Inhibitoren, Inhibitoren der Cholesterinbiosynthese, Inhibitoren der Amyloid-Aggregation, immunologische Verfahren, insbesondere mit dem A-β-Peptid oder Antikörpern gegen dieses Peptid, Verhinderung der APP-Expression, Erhöhung der APP-Clearance, Modulation der Phosphorylierung von Tau-Protein und Erniedrigung des Serumamyloid-P-Spiegels (Wolfe,Nat.Rev.Drug Discov.1 (2002) 859-866)

In der US 2002/0052407 A1, die US 6 187 756 B1 und die US 6 184 248 B1 werden Stoffgemische offenbart, die nicht steroidale anti-inflammatorische Arzneimittel (NSAID) beinhalten, zur Inhibierung anormaler Expression des Amyloid Vorläuferproteins (APP), das bekannter Weise in Zusammenhang mit Morbus Alzheimer steht. Mit Hilfe von in-vitro und in-vivo Versuchen konnte in den Beispielen gezeigt werden, dass die erfindungsgemäßen Substanzen die Überexpression von APP inhibieren. Alle drei Dokumente legen die Verwendung von nicht steroidalen anti-inflammatorischen Arzneimitteln, die nur Cyclooxygenase-2 inhibieren, nicht allerdings Cyclooxygenase-1 zur Prävention bzw. zur etwaigen Heilung von Morbus Alzheimer nahe.

Weiters werden in diesen Dokumenten ausschließlich Cyclooxygenase-2 Inhibitoren beansprucht. Oxicame und im spezielleren Lornoxicam oder analoge Verbindungen sind darin nicht offenbart.

In der WO 93/24115 A1 wird eine Methode zur Behandlung von Demenz, insbesondere von Morbus Alzheimer durch Verabreichung von nicht steroidalen anti-inflammatorischen Arzneimitteln beschrieben. Dabei wurden neben einer Reihe anderer Substanzen auch Oxicame, insbesondere Piroxicam, Isoxicam und Sudoxicam, nicht jedoch Lornoxicam oder analoge Verbindungen vorgeschlagen.

In der WO 01/78721 A1 wird eine Methode zur Prävention bzw. zur Heilung von Morbus Alzheimer durch Verabreichung von Substanzen, die den Gehalt des Amyloid-β-Polypeptids (Aβ) Aβ₄₂ reduzieren, beschrieben. Da eine hohe Expressionsrate von Aβ₄₂ für die Entstehung von Morbus Alzheimer verantwortlich gemacht wird, kann durch die Reduktion dieses Polypeptids der Krankheitsverlauf positiv beeinflusst werden. Weiters wird in diesem Dokument ein Versuch beschrieben, in dem der Einfluss von verschiedenen NSAIDs auf die Expressionsraten zweier Aß-Polypeptiden, Aβ₄₀ und Aβ₄₂, gezeigt wird. Daraus ist zu entnehmen, dass Oxicame, insbesondere Meloxicam, Peroxicam, Isoxicam und Tenoxicam (Lornoxicam oder analoge Verbindungen hierzu werden hier nicht erwähnt) die Expression von Aβ₄₂ nicht beeinflussen bzw. deren Expression sogar steigern. Aus diesem Dokument ließe sich für den Fachmann daher ableiten, dass sich Oxicame für die Verwendung zur Reduktion von Aβ₄₂ als nicht vorteilhaft erwiesen haben.

Aus der US 2002/0119193 A1 gehen pharmazeutische Zusammensetzungen hervor, die unter anderem selektive Cyclooxygenase-2 Inhibitoren beinhalten und zur Behandlung von verschiedenen Krankheiten, darunter auch Morbus Alzheimer, eingesetzt werden. Gemäß diesem Dokument weisen die erfindungsgemäßen Cyclooxygenase-2 Inhibitoren Vorteile gegenüber den herkömmlichen nicht steroidalen anti-inflammatorischen Wirkstoffen auf. Somit führt auch die Lehre von diesem Dokument von der Verwendung von Oxicamen, die sowohl Cyclooxygenase-1 als auch Cylooxygenase-2 inhibieren, weg.

Keines der bislang beschriebenen Konzepte hat allerdings einen tatsächlichen Durchbruch bei der effizienten Behandlung und vor allem Vorbeugung von AE ermöglicht. Es besteht daher immer noch ein dringender Bedarf an medikamentösen Behandlungs- und Präventionsmaßnahmen für AE.

Demgemäß betrifft die vorliegende Erfindung die Verwendung von Lornoxicam oder Lornoxicam-Analoga, die zur Herstellung einer pharmazeutischen Zusammensetzung zur Behandlung oder Verhinderung der Alzheimer'schen Erkrankung (AE) oder der Arteriosklerose.

Erst vor kurzem wurde entdeckt, dass periphere Blutplättchen die primäre Quelle des - in den cerebralen Plaques niedergeschlagenen - β-42-Peptids sind. Mit dieser Erkenntnis ist die AE als eine vaskuläre Erkrankung zu definieren. Die neue ätiologische Interpretation wird durch die Ergebnisse klinischer Studien unterstützt, welche zeigen, dass non-steroidale anti-inflammatorische Arzneimittel (NSAIDs) Symptome der AE lindern. Diesen Studien wiederum lag aber die Hypothese zugrunde, dass die AE eine entzündliche Erkrankung des Gehirns ist, wobei wiederum verschiedene Ursachen für die Entzündungserscheinungen erwähnt sind. So konnte durch Gabe von 100 - 150 mg Indometacin täglich der kognitive Abbau um etwa 9% während 6 Monaten reduziert werden. Ebenso ist bekannt, dass NSAIDs die APP-Proteinexpression und -Prozessierung beeinflussen, indem sie die Prostaglandin E2-induzierte APP-Expression reduzieren und zugleich Veränderungen bei der Spaltung von APP bewirken.

In Studien mit Ibuprofen und Indometacin konnte gezeigt werden, dass der Anteil des amyloiden β-42-Peptids gesenkt und jener des nicht amyloidogenen löslichen APP erhöht wird. Aus der Tatsache, dass Blutplättchen eine primäre Quelle der Proteine in amyloiden Plaques sind, ergibt sich in gleicher Weise ein Bezug zu arteriosklerotischen Erkrankungen.

Die durch NSAID bewirkte Reduktion der APP-Expression in Blutplättchen, die Veränderung der Spaltung des Plättchen-APP mit einer Reduktion des Plasma A-β und einem Anstieg des nicht amyloidogenen löslichen APP, die Reduktion der amyloiden Plaques in den cerebralen Gefäßen und die verminderte cerebrovaskuläre Hypoperfusion sind auch hier die grundlegenden Mechanismen des therapeutischen Effektes.

Die vorliegende Erfindung beruht daher auf dem Gedanken, dass der sporadischen AE eine vaskuläre Störung zugrunde liegt und die primäre Quelle der cerebrovaskulären Ablagerung von β-42 die peripheren Blutplättchen sind.

Mit der erfindungsgemäßen Verwendung wird die Expression von APP in Blutplättchen selektiv unterbunden und die Spaltung von Plättchen-APP beeinflusst, wobei der Plasma A-β-Spiegel fällt und der Spiegel an nicht-amyloidogenem löslichen APP steigt. Aufgrund der reduzierten Plaque-Bildung in den cerebralen Gefäßen kommt es zu keiner cerebrovaskulären Hypoperfusion, wodurch die Neurodegenerierung reduziert wird.

Eine der essentiellen Voraussetzungen für die erfindungsgemäße Verwendung ist, dass die erfindungsgemäße Substanz, also Lornoxicam oder ein Lornoxicam-Analogon, beide zentrale Isoenzyme des Eicosanoidstoffwechsels, also COX-1 und COX-2 hemmt. Speziell die Tatsache, dass das Isoenzym COX-2 mit entzündlichen Vorgängen verbunden ist, hat nach bisheriger Lehrmeinung die Anwendung COX-2 selektiver gehirngängiger Arzneistoffe suggeriert. Da aber nach den oben erwähnten neuen Erkenntnissen periphere Blutplättchen, welche nur COX-1 exprimieren, die primäre Quelle des Plaque-Proteins sind, müssen erfindungsgemäß beide inhibierenden Aktivitäten bei einem effizienten Behandlungs- und Präventionsmittel vorhanden sein.

Da COX-2 im Gehirn auch konstitutiv exprimiert wird, muss in Anbetracht des peripheren Auftretens der Blutplättchen die Hirngängigkeit eines Wirkstoffes nicht nur als nicht wünschenswert, sondern im Gegenteil sogar als Nachteil erachtet werden, da die Hemmung eines konstitutiv exprimierten Enzyms in der Regel die Hemmung physiologischer Vorgänge bewirkt und damit Arzneimittelnebenwirkungen herbeiführen kann. Somit ist auch die Eigenschaft der erfindungsgemäß zu verwendenden Mittel, nicht die Blut-Hirn-Schranke zu überwinden, ein wichtiges erfindungsgemäßes Merkmal.

Die Wirksamkeit von Lornoxicam oder Lornoxicam-Analoga im Rahmen der vorliegenden Erfindung war vor allem auch in Anbetracht des Standes der Technik, insbesondere in Anbetracht der Untersuchungsergebnisse in der WO 01/78721 A1, überraschend, da die dort beschriebenen Oxicame, insbesondere Meloxicam, Peroxicam, Isoxicam und Teroxicam die Expression von Aβ₄₂ entweder nicht beeinflussen konnten oder gar noch gesteigert haben.

Gemäß der vorliegenden Erfindung haben sich aber Lornoxicam und seine Analoga als äußerst vorteilhaft bei der Behandlung und vor allem Prävention bzw. Verlangsamung von AE und Arteriosklerose erwiesen. Unter "Lornoxicam-Analoga" werden im Rahmen der vorliegenden Erfindung alle Substanzen angesehen, die - abgeleitet von der Lornoxicam-Struktur - in den peripheren Blutplättchen den selben prinzipiellen oder vergleichbaren Effekt im Hinblick auf Aβ-42 wie Lornoxicam aufweisen und eben
- Cyclooxygenase-1 und Cyclooxygenase-2 (COX-1 und COX-2) in hibieren,
- die Blut/Hirn-Schranke unter physiologischen Bedingungen nicht überwinden können und
- die Prostaglandin E2-induzierte Induktion des Amyloid-Vor läufer-Proteins (APP) reduzieren.

Beispiele für derartige Lornoxicam-Analoga sind Enolether von 6-Chlor-4-hydroxy-2-methyl-N-(2-pyridyl)-2H-thieno (2,3-e)-1,2-thiazin-3-carbonsäureamid-1,1-dioxid (wie sie in der EP 0 313 935 A1 beschrieben und insbesondere mit der EP 0 313 935 B1 beansprucht werden), 4-Hydroxy-2-methyl-3-(2-pyridylcarbamoyl)-6-trifluormethyl-2H-thieno[2,3-e]-1,2 thiozin-1,1-dioxid (wie in der EP 0 103 142 A1 beschrieben und insbesondere in der EP 0 103 142 B1 beansprucht); Thienothiazinderivate gemäß der EP 0 001 113 A1 (B1) mit der allgemeinen Formel I 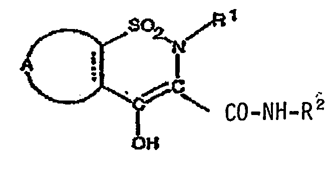 worin A mit den beiden Kohlenstoffatomen des Thiazinringes die Gruppe 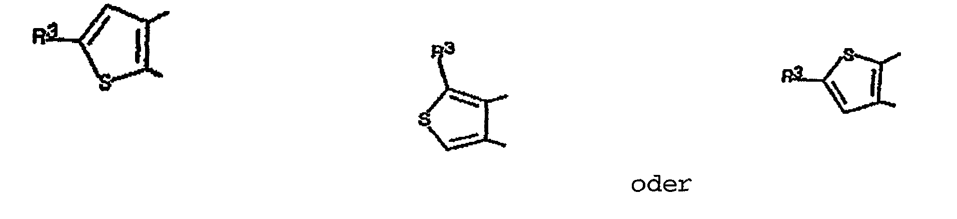 bildet und die gestrichelte Linie die im ersten und letzten Fall vorliegende Doppelbindung anzeigt, R¹ niederes Alkyl bedeutet, R² 2-Thiazolyl, 4-Methyl-2-thiazolyl., 4,5-Dimethyl-2-thiazolyl, 5-Methyl-1,3,4-thiadiazolyl, 2-Pyrazinyl, 2-Pyrimidinyl, 1,2,4-Triazin-3-yl, 2-Pyridyl, 3-Pyridyl, 4-Pyridyl, 3-Methyl-2-pyridyl, 4-Methyl-2-pyridyl, 5-Methyl-2-pyridyl, 6-Methyl-2-pyridyl, 4,6-Dimethyl-2-pyridyl, 5-Isoxazolyl, 5-Methyl-3-isoxazolyl, 3,4-Dimethyl-5-isoxazolyl, 2,6-Dimethyl-4-pyrimidinyl, 1,2,3,4-Tetrazol-5-yl oder einen gegebenenfalls durch Halogen, Hydroxy, niederes Alkyl. Trifluormethyl oder niederes Alkoxy substituierten Phenylrest bedeutet, und R³ Halogen bedeutet, sowie Salze davon, wobei der Ausdruck "niederes Alkyl" geradkettige oder verzweigte gesättigte Kohlenwasserstoffgruppen mit 1-4 Kohlenstoffatomen, wie z.B. Methyl, Ethyl, Propyl, Isopropyl und t-Butyl bezeichnet; der Ausdruck "niederes Alkoxy" sich auf Hydrocarbonoxygruppen mit bis zu 4 C-Atomen bezieht und die Bezeichnung "Halogen" sich auf die 4 Halogene Chlor, Brom, Fluor, Jod bezieht; besonders bevorzugt bedeutet dabei R³ Chlor oder Brom, wobei Chlor besonders bevorzugt ist; R¹ bedeutet vorzugsweise die Methylgruppe; R² stellt vorzugsweise 2-Thiazolyl, 5-Isoxazolyl oder 2-Pyridyl dar; A ist vorzugsweise die Gruppe

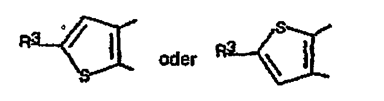

Weitere bevorzugte Beispiele der erfindungsgemäßen Lornoxicam-Analoga sind Gegenstände der AT 400 567 B und der AT 400 437 B, insbesondere die in den Ansprüchen 2, 3, 4, 5, 6, 7, 8, 9, 10, 11 oder 12 der AT 400 567 B und die in den Ansprüchen 2, 3 oder 4 der AT 400 437 B beschriebenen Substanzen. Weitere bevorzugte Lornoxicam-Analoga gemäß der vorliegenden Erfindung sind in der EP 0 657 459 A1 geoffenbart, insbesondere die in den Ansprüchen 2, 3 und 4 beanspruchten Substanzen.

Pharmazeutisch verwendbare Salze sind jene starker anorganischer Säuren, wie z.B. Salzsäure, Bromwasserstoffsäure, Schwefelsäure usw., aber auch solche organischer Säuren wie z.B. Fumarsäure, Zitronensäure, Sebacinsäure usw.

Bevorzugter Weise soll die Verabreichung der Substanz keine schweren unerwünschten Ereignisse mit sich bringen, also nebenwirkungsfrei sein. Dadurch wird die vorbeugende Einnahme dieser Substanz zur Verhinderung von AE oder Arteriosklerose möglich, ohne dass der Patient mit unerwünschten Nebenreaktionen konfrontiert ist. Die Vorbeugung ist vor allem für Personen indiziert, bei welchen ein besonderes Risiko für AE oder Arteriosklerose besteht, sei es durch genetisch bedingte Umstände (familiäre Anhäufung solcher Erkrankungen) oder durch andere Parameter. Für die Definition der Nebenwirkungsfreiheit gemäß der vorliegenden Erfindung können die Definitionen aus den einschlägigen pharmazeutischen Lehrbüchern und Standardwerken herangezogen werden. Beispielsweise sollten die Nebenwirkungen bei Anwendungsbeobachtungen unter 1 %, vorzugsweise unter 0,5 %, noch bevorzugter unter 0,1 %, insbesondere unter 0,05 %, liegen. Gegebenenfalls können die erfindungsgemäßen Substanzen mit weiteren Arzneistoffen kombiniert werden, insbesondere solche, welche etwaigen negativen Effekten, wie z.B. Magenschleimhaut-initiierende Eigenschaften, entgegenwirken (z.B. Antacida, H2-Rezeptor-Antagonisten, Protonenpumpen-Hemmer, ...). Dies ist vor allem bei Langzeitanwendungen zu berücksichtigen.

Besonders bevorzugte Substanzen zur Verwendung im Rahmen der vorliegenden Erfindung sind neben Lornoxicam vor allem 6-Chlor-4-(1-(ethoxycarbamoyloxy)ethoxy)-2-methyl-N-(2-pyridyl)-2H-thieno-(2,3-e)-1,2-thiazin-3-carbonsäureamid-1,1-dioxid und 6-Chlor-4-hydroxy-2-methyl-3-(2-pyridyl-carbamoyl)-2H-thieno [3,2-e]1,2-thiazin-1,1-dioxid.

Der besondere erfindungsgemäße Vorteil der Anwendung von Lornoxicam sowie der Lornoxicam-Analoga ergibt sich aus einer ganz besonders vorteilhaften Kombination der pharmakodynamischen und pharmakokinetischen Eigenschaften dieses Wirkstoffes.

Die pharmakodynamischen erfindungsbedingenden Besonderheiten von Lornoxicam bzw. der Lornoxicam-Analoga gemäß der vorliegenden Erfindung sind folgende:

Eine besonders wichtige Eigenschaft, welche die besondere erfindungsgemäße Eignung von Lornoxicam bedingt, liegt in der Tatsache, dass der Wirkstoff beide zentralen Isoenzyme des Eicosanoidstoffwechsels, nämlich die COX-1 und die COX-2, hemmt. Die nunmehr feststehende Tatsache, dass periphere Blutplättchen, welche nur COX-1 exprimieren, die primäre Quelle des Plaque-Proteins sind, bedingt die Überlegenheit der erfindungsgemäßen Anwendung von Lornoxicam bzw. dessen Analoga, welches eine signifikante Hemmung der COX-1 aufweist.

Lornoxicam ist des Weiteren ein Wirkstoff mit besonders hoher intrinsischer Aktivität. Da eine Therapie zur Prävention der AE bzw. von Arteriosklerose über längere Zeit erfolgen muss, stellt eine geringe Belastung des Körpers mit Wirkstoff einen weiteren Vorteil dar.

Weiters ist Lornoxicam nicht in der Lage, die Blut-Hirn-Schranke zu überwinden (Pruss et al., 3. Interscience World Conference on Inflammation, Monte Carlo (1989), Abstract 41).

In Anbetracht der über längere zeit verlaufenden präventiven Therapie der AE mit Lornoxicam ist die kurze Plasma-Halbwertszeit von Lornoxicam ein besonderer Vorteil, weil es dadurch zu keiner Kumulierung im Blut kommt. Ebenso ist die bekannte gute gastrointestinale und sonstige Verträglichkeit des Wirkstoffes von wichtiger Bedeutung. So kommen bei einer Million Verordnungen weniger als 10 gemeldete schwere unerwünschte Ereignisse vor bzw. liegen die Nebenwirkungen bei Anwendungsbeobachtungen weit unter 0,05 % (wobei bislang sämtliche Nebenwirkungsfälle wiederhergestellt werden konnten).

Die genannte Kombination pharmakodynamischer und pharmakokinetischer Eigenschaften des Wirkstoffes Lornoxicam in einer zum Zwecke der Therapie der AE und von arteriosklerotischen Erkrankungen besonders günstigen Konstellation findet sich bei keinem anderen bisher bekannt gewordenen Wirkstoff in vergleichbarer Weise.

Die Erfindung wird anhand der nachfolgenden Beispiele sowie der Zeichnungsfiguren näher erläutert.

Es zeigen:

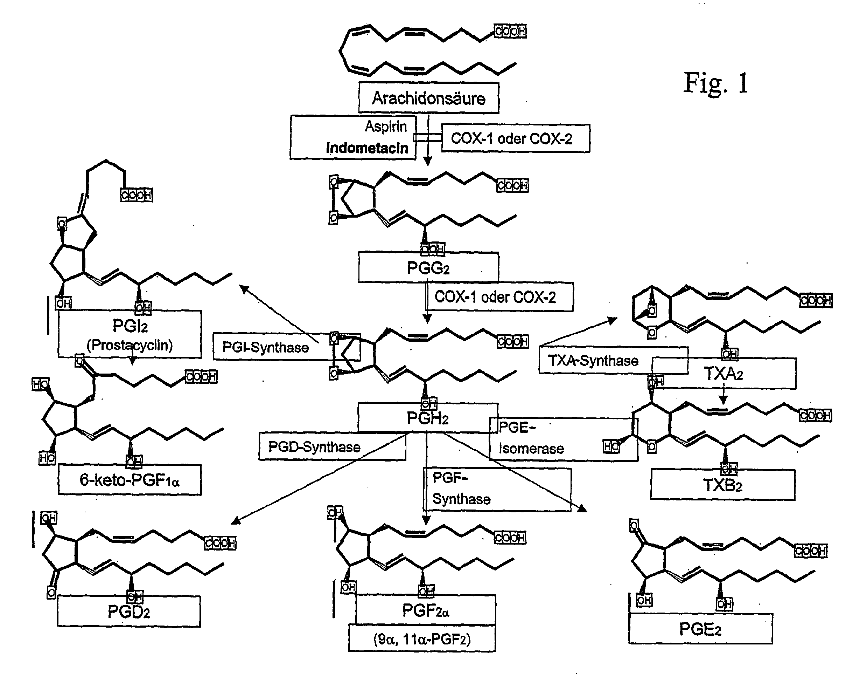

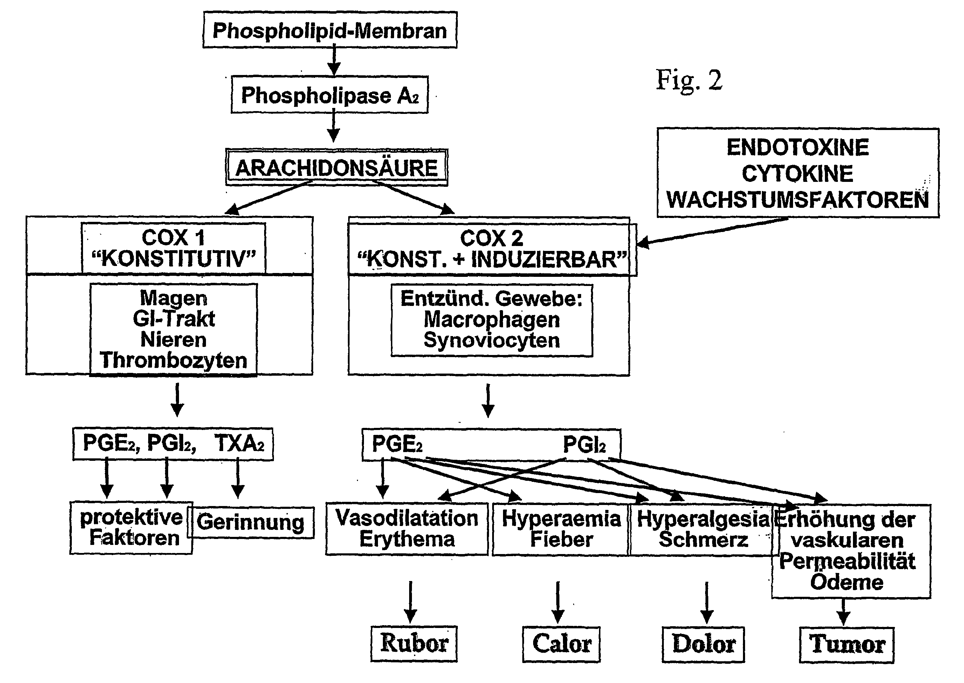

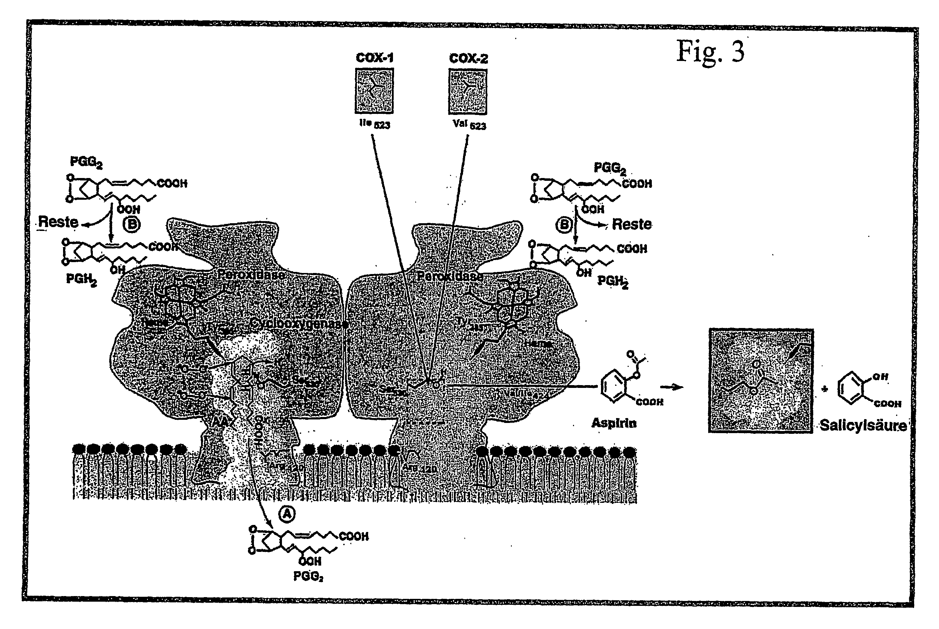

Fig. 1, 2 und 3: den Arachidonsäure-Weg und dessen Zusammenhang mit COX-1 und COX-2;

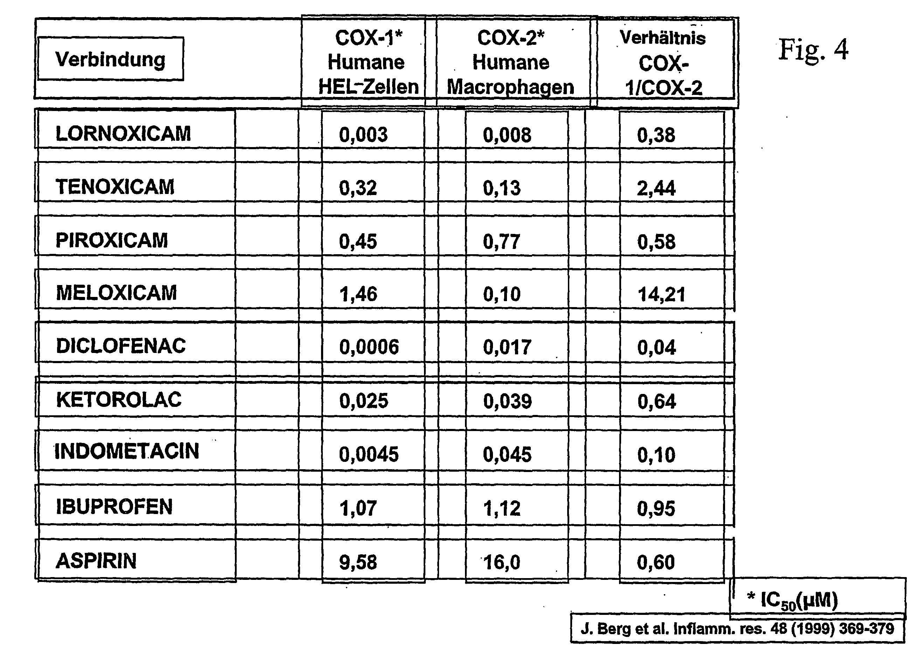

Fig. 4: die COX-inhibierende Wirkung von einigen Substanzen;

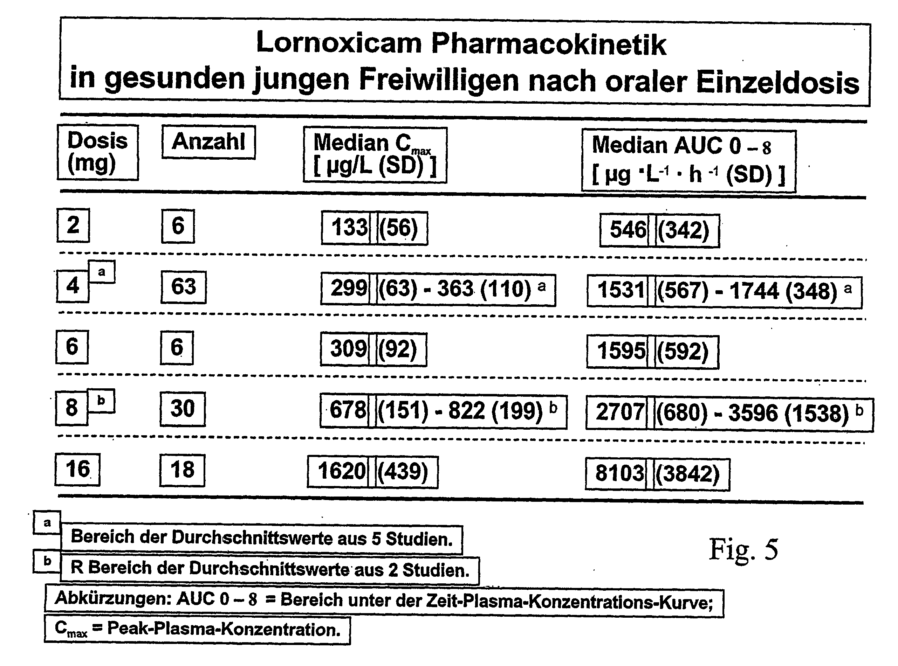

Fig. 5: die Lornoxicam-Pharmakokinetik in gesunden jungen Freiwilligen nach oraler Einzeldosis;

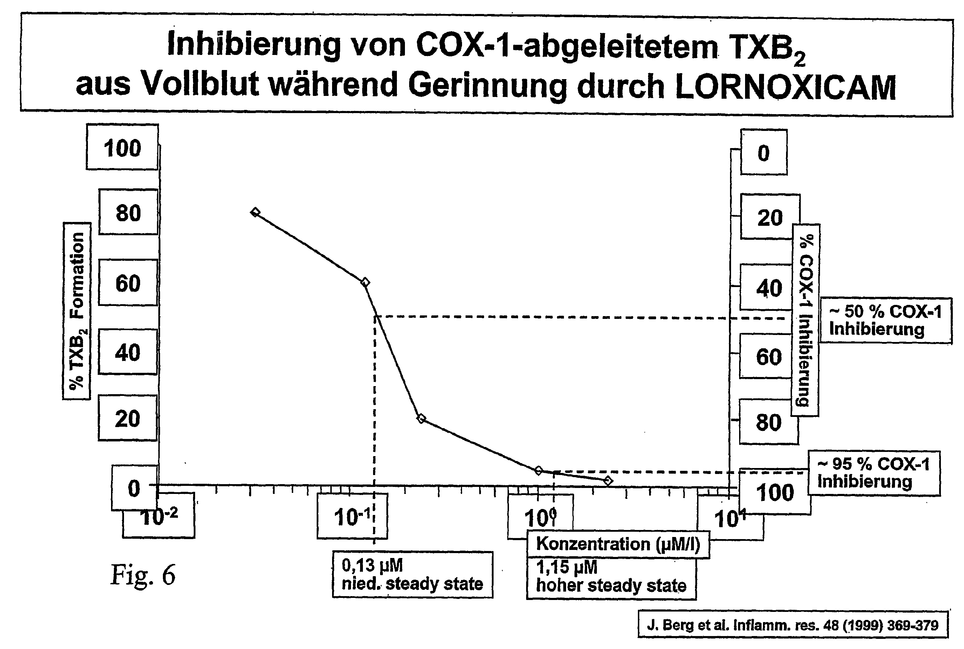

Fig. 6: die Inhibierung von COX-1-abgeleitetem TXB2 aus Vollblut während Gerinnung durch Lornoxicam;

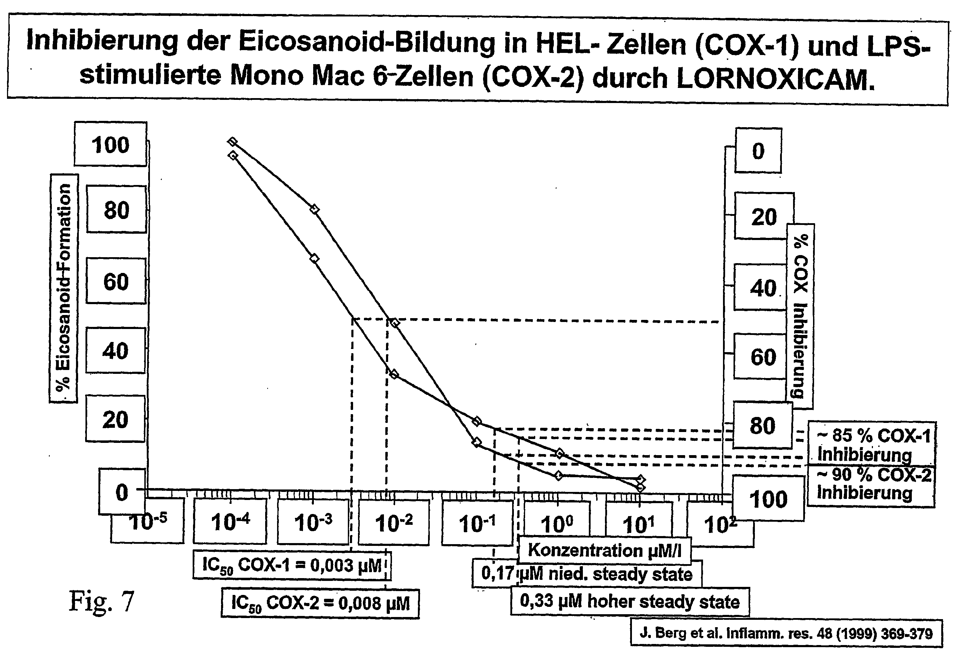

Fig. 7: die Inhibierung der Eicosanoid-Bildung in HEL-Zellen (COX-1) und LPS-stimulierte Mono Mac 6-Zellen (COX-2) durch Lornoxicam.

### Beispiele:

### Beispiel 1: Wirkung von Lornoxicam auf das APP-Processing in vitro und in vivo

### 1.1. Charakterisierung eines spezifischen Einflusses auf die APP-Spaltung in vitro:

### 1.1.1. Einfluss von Lornoxicam auf APP-Processing in neuronaler Zelllinie (SH-SY5Y)

Freisetzung des amyloiden β-42-Peptids (erniedrigt durch NSAIDs)
Freisetzung des nicht-amyloiden sAPPα-Proteins (erhöht durch NSAIDs)
Expression des APP-Holoproteins (erniedrigt durch NSAIDs)

### 1.2. Charakterisierung humaner Thrombozyten als peripheres Modell für die Wirkung von Lornoxicam auf Nervenzellen

In peripheren Thrombozyten von Morbus Alzheimer-Patienten wurde nachgewiesen, dass das nicht-amyloide sAPPα-Protein ebenfalls vermindert produziert wird (Colciaghi et al., Mol. Med. 8 (2002), 67-74). Die Expression des nicht-amyloiden sAPPα kann durch NSAIDs in Nervenzellen erhöht werden (Avramovich et al., J. Biol. Chem. 277 (2002), 31466-73). Die Ergebnisse dieser beiden Studien lassen erwarten, dass das nicht-amyloide sAPPα-Protein sich gut eignet, um die spezifische Wirkung von Lornoxicam peripher zu beobachten.

### 1.2.1. Einfluss von Lornoxicam auf APP-Processing in humanen Thrombozyten in vitro

- Freisetzung des nicht-amyloiden sAPPα-Proteins aktivierter Thrombozyten

### 1.2.2. Einfluss von Lornoxicam auf APP-Expression humaner Thrombozyten von Morbus Alzheimer-Patienten in vivo

- Expression des APP-Holoproteins
   (Dieser Parameter ist aussagekräftig, falls Lornoxicam keinen spezifischen Einfluss auf APP-Processing hat, da APP-Holoprotein im frontalen Cortex erhöht exprimiert wird (Golde et al., Neuron 4 (1990), 253-267) und NSAIDs dessen Expression vermindern können. In einer laufenden Studie wurde ebenfalls erhöhte APP-Holoprotein-Expression an Thrombozyten von Alzheimer-Patienten nachgewiesen)
- APP-Verhältnis der Thrombozyten von Morbus Alzheimer-Patienten vor und nach Lornoxicam-Behandlung
- Freisetzung des nicht-amyloiden sAPPα-Proteins aktivierter Thrombozyten

Ziel dieser Untersuchungen ist das in vivo-Monitoring an peripheren Blutthrombozyten für die postulierte Wirkung von Lornoxicam an Nervenzellen.

### 2.2. Protein-Profiling (Proteomics) humaner Thrombozyten nach in vivo-Gabe von Lornoxicam

Vergleich des Thrombozyten-Proteoms von Morbus Alzheimer und gesunden Probanden vor und nach Lornoxicam-Medikation.

Ziel dieser Untersuchungen ist das in vivo-Protein-Profiling der Wirkung von Lornoxicam an humanen Thrombozyten.

### 2.3. Einfluss von Lornoxicam auf Pharmacoproteomics humaner Thrombozyten in vitro: Protein-Profiling (Proteomics) humaner Thrombozyten nach in vitro-Inkubation mit Lornoxicam

Vergleich des Thrombozyten-Proteoms von mit Lornoxicam behandelten mit unbehandelten Kontrollthrombozyten.

Ziel dieses Beispiels ist die Charakterisierung der Thrombozytenproteine, die unmittelbar von Cyclooxygenase-Inhibitor Lornoxicam betroffen sind.

## Patentansprüche

1. Verwendung von Lornoxicam oder Lornoxicam-Analoga, die
- Cyclooxygenase 1 und Cyclooxygenase 2 (COX 1 und COX 2) inhibieren,
- die Blut/Hirn-Schranke unter physiologischen Bedingungen nicht überwinden können, und
- die Prostaglandin E2-induzierte Induktion des Amyloid-Vorläufer-Proteins (APP) reduzieren,
zur Herstellung einer pharmazeutischen Zusammensetzung zur Behandlung oder Verhinderung der Alzheimer'schen Erkrankung (AE) oder der Arteriosklerose.

2. Verwendung nach Anspruch 1, **dadurch gekennzeichnet, dass** die Verabreichung der Substanz in unter 1%, vorzugsweise unter 0,5%, noch bevorzugter unter 0,1%, insbesondere unter 0,05% der Patienten schwere unerwünschte Nebenwirkungen mit sich bringt.

3. Verwendung nach Anspruch 1 oder 2, **dadurch gekennzeichnet, dass** das Lornoxicam-Analogon ausgewählt ist aus der Gruppe 6-Chlor-4-(1-(ethoxycarbamoyloxy)ethoxy)-2-methyl-N-(2-pyridyl)-2H-thieno-(2,3-e)-1,2-thiazin-3-carbonsäureamid-1,1-dioxid and 6-Chlor-4-hydroxy-2-methyl-3- (2-pyridyl-carbamoyl) -2H-thieno[3,2-e]1,2-thiazin-1,1-dioxid.

## Claims

1. The use of lornoxicam or lornoxicam analogues which
- inhibit cyclooxygenase 1 and cyclooxygenase 2 (COX 1 and COX 2),
- cannot cross the blood/brain barrier under physiological conditions, and
- reduce the prostaglandin E2-induced induction of the amyloid precursor protein (APP),
for producing a pharmaceutical composition for the treatment or prevention of Alzheimer's Disease (AD) or of arteriosclerosis.

2. The use according to claim 1, **characterized in that** the administration of said substance entails severe undesired side effects in less than 1%, preferably less than 0.5%, even more preferably less than 0.1%, especially less than 0.05% of the patients.

3. The use according to claim 1 or 2, **characterized in that** the lornoxicam-analogue is selected from the group of 6-chloro-4-(1-(ethoxycarbamoyloxy)-ethoxy)-2-methyl-N-(2-pyridyl)-2H-thieno-(2,3-e)-1,2-thiazine-3-carboxylic acid amide-1,1-dioxide, and 6-chloro-4-hydroxy-2-methyl-3-(2-pyridyl-carbamoyl)-2H-thieno[3,2-e]1,2-thiazine-1,1-dioxide.

## Revendications

1. Utilisation du lornoxicam ou d'analogues du lornoxicam qui
- inhibent la cyclooxygénase 1 et la cyclooxygénase 2 (COX 1 et COX 2),
- ne peuvent pas vaincre la barrière hémato-encéphalique dans les conditions physiologiques, et
- réduisent l'induction de la protéine précurseur d'amyloïde (APP) induite par la prostaglandine E2,
pour la production d'une composition pharmaceutique pour traiter ou éviter la maladie d'Alzheimer (AE) ou l'artériosclérose.

2. Utilisation selon la revendication 1 **caractérisée en ce que** l'administration de la substance entraîne des effets secondaires indésirables graves chez moins de 1 %, de préférence moins de 0,5 %, de préférence encore moins de 0,1 %, en particulier moins de 0,05 % des patients.

3. Utilisation selon la revendication 1 ou 2 **caractérisée en ce que** l'analogue du lornoxicam est choisi dans le groupe 6-chloro-4-(1-(éthoxycarbamoyloxy)éthoxy)-2-méthyl-N-(2-pyridyl)-2H-thiéno-(2,3-e)-1,2-thiazine-3-carboxamide -1,1-dioxyde et 6-chloro-4-hydroxy-2-méthyl-3-(2-pyridyl-carbamoyl)-2H-thiéno[3,2-e]-1,2-thiazine-1,1-dioxyde.
